# EUROPEAN PATENT APPLICATION

(11) **EP 3 168 210 A1**
(43) Date of publication of application: **17.05.2017**
(21) Application number: 15194610.0
(22) Date of filing: 13.11.2015
(51) Int. Cl.: C07D 213/30, A61K 31/4406

(54) **CRYSTALLINE FORMS OF ENTINOSTAT**

(71) Applicant: SANDOZ AG, 4056 Basel (CH)
(72) Inventor: STEFINOVIC, Marijan, 6250 Kundl (AT); REECE, Hayley, Milton Bridge EH26 0BE (GB)
(74) Representative: Kluschanzoff, Harald

(57) **Abstract**

Entinostat is a histone deacetylase inhibitor undergoing clinical investigation in multiple types of solid tumors, such as breast cancer, and hematologic cancers. Crystalline form D and E of Entinostat and crystal form A in high crystal purity are provided. Crystalline form D can be obtained in high chemical purity, exhibits improved water solubility and allows efficient purification of Entinostat with removal of coloured impurities. Processes for the preparation of such crystalline forms and of form A with improved chemical and crystal purity are also provided.

## Description

### FIELD OF INDUSTRIAL APPLICABILITY

The present invention relates to polymorphic forms of Entinostat, processes for their preparation, compositions comprising them and their medical use.

### BACKGROUND OF THE DISCLOSURE

Histone deacetylase (HDAC) inhibitors are an emerging class of therapeutic agents that promote differentiation and apoptosis in hematologic and solid malignancies through chromatin remodeling and gene expression regulation. Entinostat ((pyridin-3-yl)methyl 4-(2-aminophenylcarbamoyl)benzylcarbamate) is a benzamide HDAC inhibitor undergoing clinical investigation in multiple types of solid tumors, such as breast cancer, and hematologic cancers. Its chemical preparation is disclosed in example 48 of EP 847992 A1 and its chemical structure is reported below:

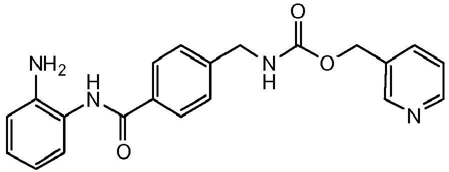

WO 2009/076206 A1 discloses a process for the preparation of Entinostat. The solid form of the obtained product is not disclosed. Reproduction of such process affords a mixture of crystal forms A and B, as disclosed in WO 2010/022988 A1.

WO 2010/022988 A1 describes crystalline forms A, B and C of Entinostat and processes for the preparation of crystalline forms A and B. As disclosed therein, crystal form A is difficult to obtain in high chemical purity and recrystallization from common solvents, such as ethanol, methanol and acetonitrile, does not afford a chemically pure substance. It is further disclosed that crystal form A, not being the thermodynamically most stable form, is difficult to obtain in polymorphic pure form. Form B should allegedly solve these problems; however example 1 of WO 2010/022988 A1 discloses that 15 kg of charcoal for 30 kg of Entinostat are needed in order to achieve the desired purification. The use of such large amount of charcoal is unusual in the art and may lead to loss of product absorbed on the charcoal as well as to the production of unnecessary waste. Indeed the reported yield of such example is only 30-80% of theory.

An object of the present invention is thus the provision of a crystalline form of Entinostat that allows its efficient chemical purification, as well as the provision of such a purification process. A further object is the provision of a process for the preparation of crystalline form A with enhanced chemical and polymorphic purity and free of coloured impurities.

Entinostat is sparingly soluble in water and its low solubility can make the preparation of pharmaceutical compositions having adequate dissolution challenging. A further object of the present invention is thus the provision of a solid form of Entinostat having improved water solubility and the provision of a pharmaceutical composition of Entinostat exhibiting improved dissolution in water.

### SUMMARY OF THE DISCLOSURE

The present disclosure provides crystalline forms D and E of Entinostat and a process for their preparation. It also provides a process for the purification of Entinostat form A by crystallization and isolation of Entinostat crystal form D and E. Crystal form D can be obtained in high chemical purity, exhibits improved water solubility and allows efficient purification of Entinostat with removal of coloured impurities.

### BRIEF DESCRIPTION OF THE DRAWINGS

FIG. 1 illustrates the x-ray powder diffraction patterns of crystalline form D.
FIG. 2 illustrates the x-ray powder diffraction patterns of crystalline form E.
FIG. 3 illustrates the DTA/TGA trace of crystalline form D.
FIG. 4 illustrates the DTA/TGA trace of crystalline form E.

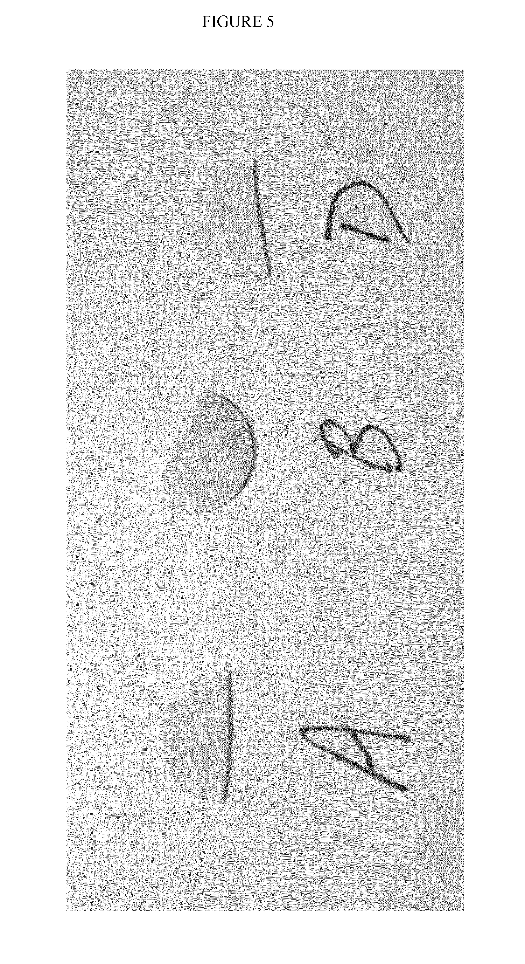

FIG. 5 compares the colours of crystal forms A, B and D after compression.
FIG. 6 compares the x-ray powder diffraction patterns of form A and of the material obtained from reference example 1

### DETAILED DESCRIPTION OF THE DISCLOSURE

The disclosure relates to crystalline forms of Entinostat, which are described and characterized herein.

### Definitions

As used herein "polymorph" refers to crystalline forms having the same chemical composition but different spatial arrangements of the molecules, atoms, and/or ions forming the crystal.

As used herein "solvate" refers to a crystalline form of a molecule that further comprises molecules of solvent incorporated into the crystalline lattice structure. The solvent molecules in the solvate may be present in a regular arrangement and/or a non-ordered arrangement. The solvate may comprise either a stoichiometric or nonstoichiometric amount of the solvent molecules. For example, a solvate with a nonstoichiometric amount of solvent molecules may result from partial loss of solvent from the stoichiometric solvate. Solvates may occur as dimers or oligomers comprising more than one molecule or Entinostat within the crystalline lattice structure.

As used herein, the term "substantially pure" with reference to a particular polymorphic form means that the polymorphic form includes less than 10%, preferably less than 5%, more preferably less than 3%, most preferably less than 1% by weight of any other physical forms of the compound.

The term "essentially the same" with reference to X-ray diffraction peak positions means that typical peak position and intensity variability are taken into account. For example, one skilled in the art will appreciate that the peak positions (2θ) will show some inter-apparatus variability, typically as much as 0.2°. Further, one skilled in the art will appreciate that relative peak intensities will show inter-apparatus variability as well as variability due to degree of crystallinity, preferred orientation, prepared sample surface, and other factors known to those skilled in the art, and should be taken as qualitative measure only.
Consequently, it is to be understood that the crystal forms of the present invention are not limited to the crystal forms that provides X-ray diffraction patterns completely identical to the X-ray diffraction patterns depicted in the accompanying figures disclosed herein. Any crystal forms that provide X- ray diffraction patterns substantially identical to those disclosed in the accompanying Figures fall within the scope of the present invention. The ability to ascertain substantial identities of X-ray diffraction patterns is within the purview of one of ordinary skill in the art.

The term "slurry", as used herein, means a saturated solution of the compound, which may also contain an additional amount of the compound to afford a heterogeneous mixture of the compound and a solvent at a given temperature.

### Crystal form D

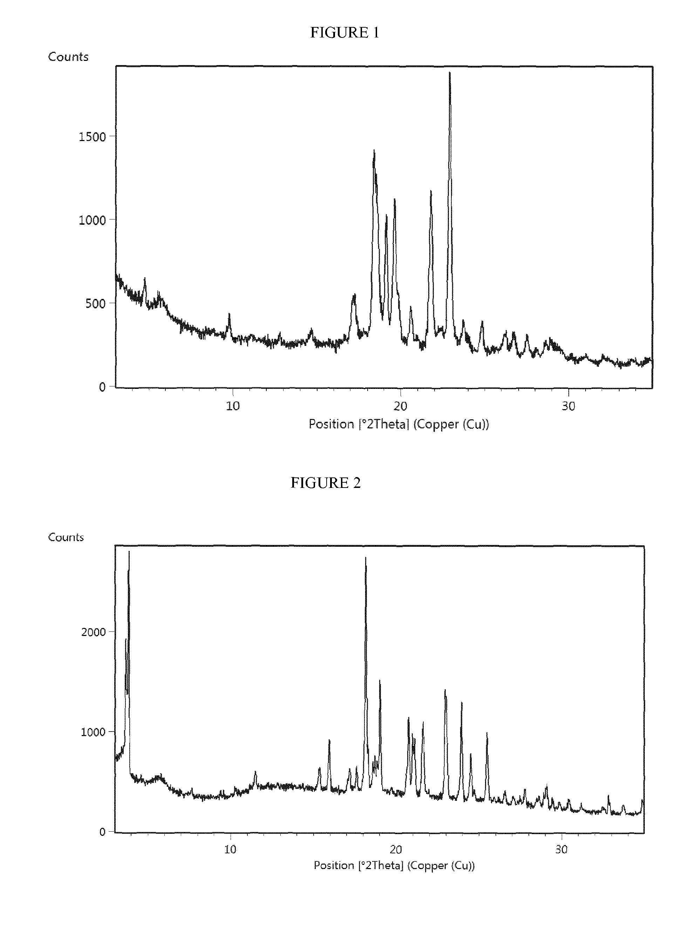

In one embodiment, the present invention provides a crystalline form of Entinostat having an X-ray diffraction pattern substantially the same as the X-ray powder diffraction pattern shown in FIG. 1.

Crystalline form D may be characterized by a X-ray powder diffraction pattern (XRPD) comprising peaks at 2θ values of 4.8°, 9.8°, 17.2°, 19.7° and 22.9°, measured at a temperature of about 20°C and using Cu-Kα radiation (wavelength λ= 1.5418 Å).

Preferably, crystalline form D may be characterized by a x-ray powder diffraction pattern comprising 2θ values of 4.8°, 9.8°, 12.8°, 14.7°, 17.2°, 18.4°, 19.7°, 21.8° and 22.9°, measured at a temperature of about 20°C and using Cu-Kα radiation (wavelength λ= 1.5418 Å).

The complete listing of peaks and relative intensities are reported in the following table:

| Position 2θ [°] | Relative intensity [%] |
|---|---|
| 4.8 | 5.0 |
| 5.7 | 4.7 |
| 9.8 | 6.5 |
| 12.8 | 2.7 |
| 14.7 | 4.8 |
| 17.2 | 18.3 |
| 18.4 | 70.4 |
| 19.1 | 40 |
| 19.7 | 45.3 |
| 20.6 | 13.7 |
| 21.8 | 57.4 |
| 22.4 | 8.6 |
| 22.9 | 100 |
| 23.8 | 9.7 |
| 24.8 | 10.5 |
| 26.2 | 8.4 |
| 26.8 | 8.7 |
| 27.5 | 7.2 |
| 28.7 | 5.4 |
| 29.5 | 3.8 |
| 31.0 | 2.0 |
| 32.1 | 1.9 |
| 33.8 | 1.2 |

In one embodiment, the present invention provides a crystalline form D of Entinostat having a DTA/TGA trace substantially the same as the DTA/TGA trace shown in FIG. 2. Crystalline form D may be characterized by a DTA/TGA trace showing an exotherm at 115°C and a melting endotherm at 151°C.

In a preferred embodiment, crystal form D is in substantially pure form. Preferably, Form D includes less than 10%, more preferably less than 5%, even more preferably less than 3%, most preferably less than 1% by weight of other crystalline forms, preferably selected from the group consisting of crystal form A, crystal form B and crystal form C, wherein form A is characterized by a x-ray powder diffraction pattern (XRPD) comprising peaks at 2θ values of 18.4°, 18.8°, 19.1°, 20.9°, 22.6°, 26.4°, 26.7° and 27.2°, wherein form B is characterized by a x-ray powder diffraction pattern (XRPD) comprising peaks at 2θ values of 9.2°, 18.1 °, 19.4°, 20.0°, 20.4°, 21.1°, 22.1°, 25.8° and 27.4°, wherein form C is characterized by a x-ray powder diffraction pattern (XRPD) comprising peaks at 2θ values of 17.7°, 18.4°, 18.8°, 19.2°, 20.0°, 22.0°, 22.3°, 23.2° and 23.4°, all peaks measured at a temperature of about 20°C and using Cu-Kα radiation (wavelength λ= 1.5418 Å).

### Crystal form E

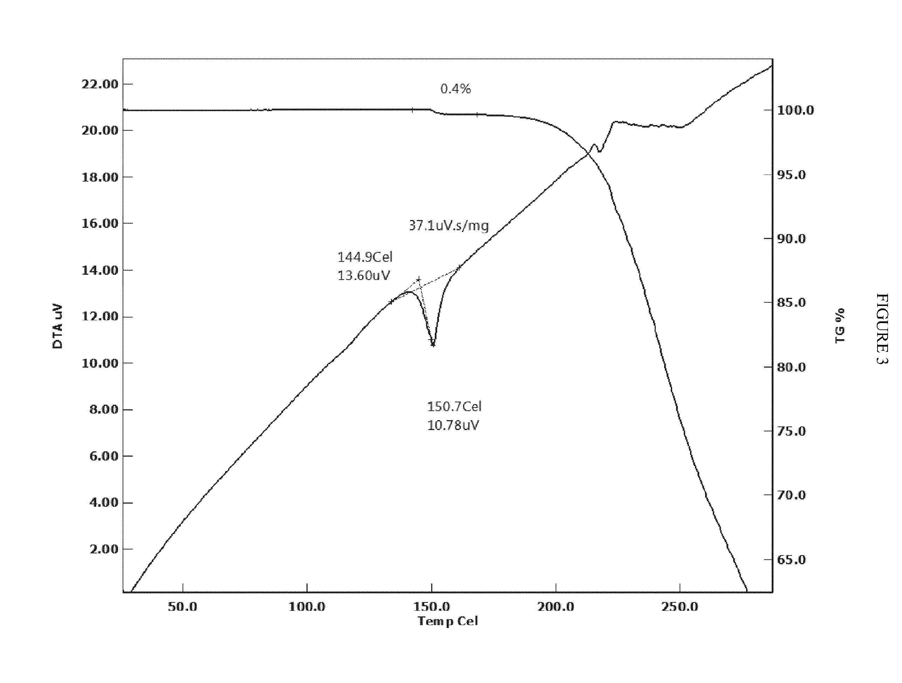

In one embodiment, the present invention provides a crystalline form E of Entinostat having an X-ray diffraction pattern substantially the same as the X-ray powder diffraction pattern shown in FIG. 3.

Crystalline form E may be characterized by a X-ray powder diffraction pattern (XRPD) comprising peaks at 2θ values of 3.8°, 15.3° and 21.6°, measured at a temperature of about 20°C and using Cu-Kα radiation (wavelength λ= 1.5418 Å).

Preferably, crystalline form E may be characterized by a x-ray powder diffraction pattern comprising 2θ values of 3.8°, 15.3°, 18.2°, 21.6°, 23.9° and 24.5°, measured at a temperature of about 20°C and using Cu-Kα radiation (wavelength λ= 1.5418 Å).

The complete listing of peaks, their heights and relative intensities are reported in the following table:

| Position 2θ [°] | Relative intensity [%] |
|---|---|
| 3.8 | 100.00 |
| 5.8 | 14.39 |
| 7.6 | 3.96 |
| 11.5 | 9.21 |
| 15.3 | 11.67 |
| 15.9 | 21.50 |
| 17.1 | 11.84 |
| 18.2 | 97.82 |
| 19.0 | 42.91 |
| 20.9 | 16.52 |
| 21.6 | 44.12 |
| 23.0 | 64.35 |
| 23.9 | 42.29 |
| 24.5 | 23.55 |
| 25.5 | 33.34 |
| 26.6 | 7.22 |
| 27.7 | 8.66 |
| 28.6 | 6.41 |
| 29.1 | 15.19 |
| 30.4 | 5.45 |
| 31.2 | 3.54 |
| 32.5 | 3.19 |
| 33.7 | 4.17 |

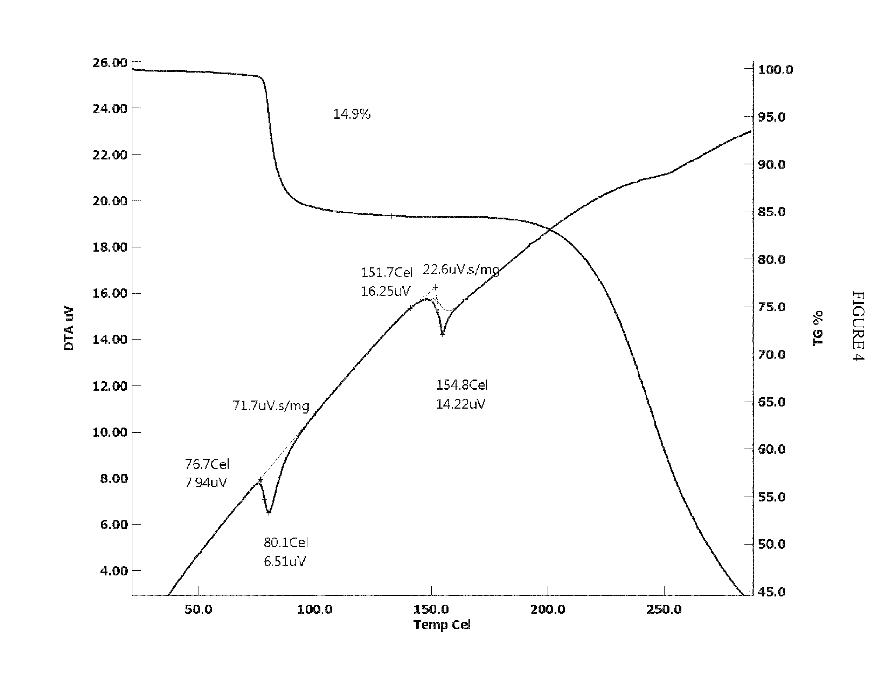

In one embodiment, the present invention provides a crystalline form E of Entinostat having a DTA/TGA trace substantially the same as the DTA/TGA trace shown in FIG. 4. Crystalline form E may be characterized by a DTA/TGA trace showing an endotherm at 80°C and a melting endotherm at 155°C. Crystal form E is a 1,4-dioxane solvate, preferably in a 1:1 Entinostat / dioxane ratio.

In a preferred embodiment, crystal form E is in substantially pure form. Preferably, Form E includes less than 10%, more preferably less than 5%, even more preferably less than 3%, most preferably less than 1% by weight of other crystalline forms, preferably selected from the group consisting of crystal form A, crystal form B and crystal form C, wherein form A is characterized by a x-ray powder diffraction pattern (XRPD) comprising peaks at 2θ values of 18.4°, 18.8°, 19.1°, 20.9°, 22.6°, 26.4°, 26.7° and 27.2°, wherein form B is characterized by a x-ray powder diffraction pattern (XRPD) comprising peaks at 2θ values of 9.2°, 18.1 °, 19.4°, 20.0°, 20.4°, 21.1°, 22.1°, 25.8° and 27.4°, wherein form C is characterized by a x-ray powder diffraction pattern (XRPD) comprising peaks at 2θ values of 17.7°, 18.4°, 18.8°, 19.2°, 20.0°, 22.0°, 22.3°, 23.2° and 23.4°, all peaks measured at a temperature of about 20°C and using Cu-Kα radiation (wavelength λ= 1.5418 Å).

### Crystal form A in substantially pure form

In one embodiment, the present invention provides a crystalline form A of Entinostat in substantially pure form. Form A may be characterized by a x-ray powder diffraction pattern (XRPD) comprising peaks at 2θ values of 18.4°, 18.8°, 19.1°, 20.9°, 22.6°, 26.4°, 26.7° and 27.2°, measured at a temperature of about 20°C and using Cu-Kα radiation (wavelength λ= 1.5418 Å).

Preferably, Form A includes less than 10%, more preferably less than 5%, even more preferably less than 3%, most preferably less than 1% by weight of other crystalline forms, preferably selected from the group consisting of crystal form B and crystal form C, wherein form B is characterized by a x-ray powder diffraction pattern (XRPD) comprising peaks at 2θ values of 9.2°, 18.1 °, 19.4°, 20.0°, 20.4°, 21.1°, 22.1°, 25.8° and 27.4°, wherein form C is characterized by a x-ray powder diffraction pattern (XRPD) comprising peaks at 2θ values of 17.7°, 18.4°, 18.8°, 19.2°, 20.0°, 22.0°, 22.3°, 23.2° and 23.4°, all peaks measured at a temperature of about 20°C and using Cu-Kα radiation (wavelength λ= 1.5418 Å).

### Preparation process

One method to prepare crystalline form E of Entinostat comprises the steps of suspending and/or dissolving Entinostat in a solvent comprising dioxane, preferably in pure dioxane, and then isolating crystalline form E. The mixture may be heated to promote dissolution and the solution volume may then be reduced by evaporation to produce a slurry. The slurry can be stirred for 12 to 120 hours at room temperature and filtered to provide crystalline form E.

One method to prepare crystalline form D of Entinostat comprises the steps of drying crystalline form E of Entinostat. The drying step is preferably performed under vacuum, preferably at a temperature below 40°C, more preferably below 35°C, preferably for more than 1 week, more preferably for more than 2 weeks. Preferably, a fluidized bed dryer is used. Alternatively, a belt dryer, a rotary dryer or a vacuum tray dryer with appropriately controlled temperature, cycle time and humidity can be used. Crystalline form E can thus be used as an intermediate in the preparation of crystalline form D.

Crystalline form D of Entinostat can be conveniently converted to crystalline form A, wherein form A is characterized by a x-ray powder diffraction pattern (XRPD) comprising peaks at 2θ values of 18.4°, 18.8°, 19.1°, 20.9°, 22.6°, 26.4°, 26.7° and 27.2°, measured at a temperature of about 20°C and using Cu-Kα radiation (wavelength λ= 1.5418 Å). One method to prepare crystalline form A of Entinostat comprises the steps of contacting the crystalline form D of Entinostat of the invention with a solvent selected from the group consisting of ethanol, acetone, acetonitrile, anisole, methyl ethyl ketone, t-butyl methyl ether, dimethylacetamide, dimethylformamide, ethyl acetate, isopropyl acetate, methanol, 2-methyltetrahydrofuran, methyl isobutyl ketone, toluene, tetrahydrofuran, 2-propanol / water mixtures, acetone / water mixtures and acetonitrile / water mixtures, and isolating crystalline form A. Preferably the solvent is selected from the group consisting of acetone and acetone / water mixtures. The Entinostat starting material may also contain crystalline form C, wherein form C is characterized by a x-ray powder diffraction pattern (XRPD) comprising peaks at 2θ values of 17.7°, 18.4°, 18.8°, 19.2°, 20.0°, 22.0°, 22.3°, 23.2° and 23.4°, measured at a temperature of about 20°C and using Cu-Kα radiation (wavelength λ= 1.5418 Å), and still lead to the desired crystalline form A.

Seed crystals may be added to any crystallization mixture to promote crystallization. Seeding may be employed to control growth of a particular polymorph or to control the particle size distribution of the crystalline product.

The crystalline Entinostat obtainable by the above mentioned processes is also an embodiment of the present invention.

### Advantages

Compression studies of crystal forms A, B and D showed all forms to maintain the same polymorphic form with a slight crystallinity decrease. However crystal form D produced an off-white disc, Form A a pale yellow disc and Form B a darker, beige coloured disc with an uneven colour distribution across the disc surface. Therefore crystal form D of the invention, unlike form A and B, allows an efficient removal of coloured impurities.

The present invention provides a way to access highly pure crystal form A of Entinostat starting from commercially available lower purity Entinostat by using crystalline form D and E as an intermediate. The procedure is carried out under near ambient temperature and does not require the use of strong acids, thereby avoiding degradation products resulting from thermal or acid mediated decomposition.

In addition crystalline form D of Entinostat provides several benefits over the known crystalline forms, since it exhibits substantially enhanced water solubility, which in turn allows the preparation of a pharmaceutical composition of Entinostat exhibiting improved dissolution in water. The water solubilities at 25°C of different crystal forms of Entinostat have been measured by HPLC and are set out in the following table:

| | Form A | Form B | Form D |
|---|---|---|---|
| Water solubility | 0.048 mg/mL | 0.028 mg/mL | 0.13 mg/mL |

### Medical use and formulations

The crystalline forms of the invention may be used in the treatment of solid tumors, including breast cancer, and hematologic cancers. They may be formulated with one or more excipients or other active pharmaceutical ingredients to provide formulations suitable for the treatment of the indications identified above. Such formulations may optionally include one or more other components selected, for example, from the group consisting of excipients, such as diluents, binders, disintegrants, lubricants, preservatives and coating materials, and other active pharmaceutical ingredients of different molecular structure. Alternatively crystalline form D may be converted to crystalline form A as described above, which can be then used for the preparation of suitable finished dosage forms.

### X-ray Powder Diffraction (XRPD)

XRPD analysis was carried out on a PANalytical X'Pert Pro X-ray Diffractometer, scanning the samples between 3 and 35 °2-theta. Material was loaded into a 96-well plate with mylar film as the base. The samples were then loaded into the plate holder of a PANalytical X'Pert Pro X-ray Diffractometer running in transmission mode and analyzed, using the following experimental conditions:

| Raw Data Origin: | XRD measurement (*.XRDML) |
|---|---|
| Scan Axis: | Gonio |
| Start Position [°2θ]: | 3.0066 |
| End Position [°2θ]: | 34.9866 |
| Step Size [°2θ]: | 0.0130 |
| Scan Step Time [s]: | 18.8700 |
| Scan Type: | Continuous |
| PSD Mode: | Scanning |
| PSD Length [°2θ]: | 3.35 |
| Offset [°2θ]: | 0.0000 |
| Divergence Slit Type: | Fixed |
| Divergence Slit Size [°]: | 1.0000 |
| Specimen Length [mm]: | 10.00 |
| Measurement Temperature [°C]: | 25.00 |
| Anode Material: | Cu |
| K-Alpha [Å]: | 1.54060 |
| K-Alpha2 [Å]: | 1.54443 |
| K-Beta [Å]: | 1.39225 |
| K-A2/K-A1 Ratio: | 0.50000 |
| Generator Settings: | 40 mA, 40 kV |
| Diffractometer Type: | 0000000011154173 |
| Diffractometer Number: | 0 |
| Goniometer Radius [mm]: | 240.00 |
| Dist. Focus-Diverg. Slit [mm]: | 91.00 |
| Incident Beam Monochromator: | No |
| Spinning: | No |

### Thermogravimetric/Differential Thermal Analysis (TG/DTA)

Approximately 5 mg of material was weighed into an open aluminium pan and loaded into a Seiko TGA6200 (simultaneous thermogravimetric/differential thermal analyser (TG/DTA)) and held at room temperature. The sample was then heated at a rate of 10°C/min from 20°C to 300°C during which time the change in sample weight was recorded along with any differential thermal events (DTA). Nitrogen was used as the purge gas, at a flow rate of 300 cm³/min.

### Compression study

The compression study was performed using a Specac hydraulic press. A sample powder (approx. 100mg) is loaded into a metal die (ring shaped metal holder). A snugly fitted billet (a cylinder-shaped metal rod) is then placed into the die, on top of the powder sample. The die and billet assembly is then placed into the hydraulic press and compressed to a pressure of 5 tonnes/cm² and held under that pressure for 1h. The billet is then removed and the disc extracted from the die.

The following non-limiting examples are illustrative of the disclosure.

### EXAMPLES

### Reference example 1

Example 48 of EP 847992 A1 was reproduced.

### Step 1

To suspension of 4-(aminomethyl)benzoic acid and triethylamine in dichloromethane (750 mL) precooled to 5°C was added trifluoroacetic anhydride within 1 hour. The mixture was stirred at a temperature < 10°C for 3 hours and then acidified with concentrated HCl (pH = 1). The resulting precipitate was filtered, washed with water and diethyl ether and dried to give the desired product as a white solid.

### Step 2

The product of Step 1 was suspended in dichloromethane (300 mL). DMF was added followed by dropwise addition of oxalyl chloride within 30 min.. Then the mixture was stirred at RT for 1 hour until completion on TLC and concentrated under reduced pressure. The above-prepared acid chloride was re-dissolved in dichloromethane (50 mL) and was added to a solution of Boc-o-phenyldiamine in pyridine (150 mL) and dichloromethane (100 mL) precooled to 5°C within 30 minutes. Then the mixture was stirred for 2 hours at RT, diluted with dichloromethane (300 mL) and a saturated solution of NaHCO₃ was added (300 mL). The layers were separated and the organic layer was washed with a saturated solution of NaHCO₃ (300 mL) and brine (300 mL). The organic layer was dried and concentrated to give a brown solid, which was suspended in diisopropyl ether (600 mL), stirred at RT for 30 minutes and filtered to give desired the product as an orange solid.

### Step 3

The product from Step 2 was suspended in methanol / water and potassium carbonate was added. The mixture was heated to 70°C and stirred overnight. Methanol was evaporated and the product was extracted with dichloromethane (2 x 500 mL). The combined organic layers were washed with saturated brine (300 mL), dried and concentrated to give the desired product as red foam.

### Step 4

To 3-pyridinemethanol dissolved in THF (467 mL) was added carbonyl diimidazole in portions within 30 minutes. The mixture was then stirred at room temperature for 3 hours until no starting material was observed on TLC. The product from Step 3, dissolved in THF (200 mL), was then added dropwise to the reaction mixture and stirring was continued overnight. The mixture was diluted with ethyl acetate (500 mL) and washed with brine (3 x 300 mL). The organic layer was dried and concentrated to give dark brown foam. The crude reaction mixture was then purified via column chromatography on SiO₂.

### Step 5

The product from Step 4 was dissolved in methanol and 4 M HCl was added dropwise within 1 hour. The mixture was stirred for 2 hours until completion on TLC and then slowly poured into beaker containing a stirred solution of 1 M NaOH (800 mL). This mixture was then diluted with dichloromethane (500 mL) and the layers were separated. The aqueous layer was then extracted with dichloromethane (2 x 250mL). The combined organic layers were washed with brine (2 x 250 mL), dried and concentrated to give desired product as light brown solid. The crude solid was recrystallized from EtOH (380 mL), cooled to 0°C and filtered, washed with cold EtOH (100 mL), dried to give Entinostat as a beige solid.

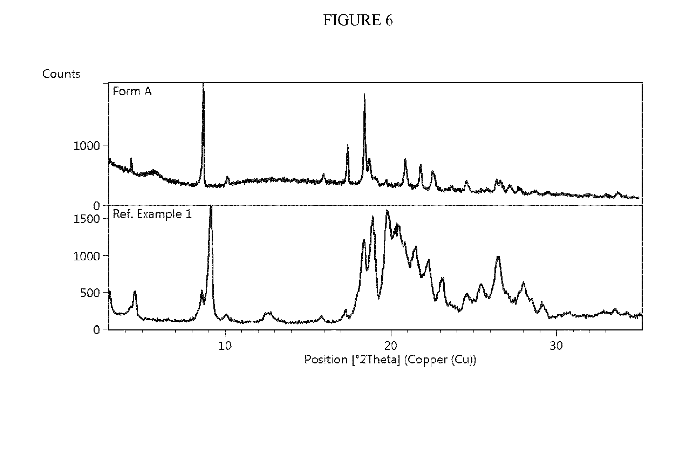

The diffractogram of the product is shown in Figure 6 and shows the presence of another crystalline phase in addition to crystal form A.

### Reference example 2

The example of WO 2009/076206 A1 was reproduced.

### Step 1

To suspension of carbonyl diimidazole in THF (214 mL) cooled to 10°C was added 3-pyridinemetanol in THF (90 mL) and the mixture was stirred at RT for 1 hour. The mixture prepared above was added dropwise to mixture of 4-(aminomethyl)benzoic acid, DBU, triethylamine in THF (415 mL) and was stirred at RT for 14 hours. TLC analysis showed no starting material. The THF was evaporated, and the mixture was diluted with water (600 mL) and was acidified while stirring to pH = 5. The resulting white precipitate was filtered, washed with water (600 ml) and methanol (150 mL) and dried to give 65.0 g of product.

### Step 2

The product from Step 1 was suspended in THF. Carbonyl diimidazole was added in portions within 30 minutes and the mixture heated to 60°C for 1.5 hours. According to TLC no starting material was detected. The mixture was cooled to room temperature and treated with o-phenyldiamine and TFA (added in one portion). The dark solution was stirred at RT for 14 hours. THF was evaporated and the residue portioned between ethyl acetate (1500 mL) and water (500 mL). The layers were separated and the water layer was extracted twice with 250 mL of ethyl acetate. The combined organics were dried and concentrated. The residue was suspended in dichloromethane (10 mL/g), stirred for 30 minutes, filtered and dried (this procedure was applied twice) resulting in77 g of Entinostat as a beige solid. The compound was analyzed by XRPD and resulted to be a mixture of form A and form B.

### Example 1

Entinostat (3 g) was dissolved in 1,4-dioxane (400 mL) and the solution volume was reduced to ca. 10 mL by evaporation to produce a slurry, which was then stirred at 22°C. The solid was then filtered and analyzed and found to be Entinostat crystalline form E. The XRPD and the DSC trace of the material are shown respectively in Figures 2 and 4.

Form E was slowly dried at 35°C for 4 weeks *in vacuo.* The resulting solid was analyzed and found to be Form D. The XRPD and the DTA/TGA trace of the material are shown respectively in Figures 1 and 3.

### Example 2

Entinostat (3 g) was dissolved in 1,4-dioxane (400 mL) and the solution filtered through a Whatman GF/F filter and evaporated to dryness. After analysis by XRPD, the solid material was found to consist of poorly crystalline Form E. 1,4-Dioxane (6 mL) was then added and the material was slurried at 22°C. The solid was then filtered and slowly dried *in vacuo* over 4 weeks at 35°C. The solid was then analyzed and found to be Entinostat crystalline form D.

### Example 3

Entinostat form D (400 mg) was suspended in acetone : water (9:1). The resulting slurry was heated to 40°C and held at that temperature for ca. 2 hours and then filtered through a 0.45 µm frit and the solution cooled to 5°C overnight. The collected solid was then dried *in vacuo* for 16 h, analyzed and found to be Entinostat crystalline form A.

### Example 4

The experiment of example 3 is repeated using a mixture of crystalline forms C and D. Crystalline form A is obtained.

## Claims

1. Crystalline form D of Entinostat having a x-ray powder diffraction pattern comprising peaks at 2θ values of 4.8°, 9.8°, 17.2°, 19.7° and 22.9°, measured at a temperature of about 20°C and using Cu-Kα radiation (wavelength λ= 1.5418 Å).

2. The crystalline form according to claim 1 further **characterized by** a x-ray powder diffraction pattern comprising 2θ values of 4.8°, 9.8°, 12.8°, 14.7°, 17.2°, 18.4°, 19.7°, 21.8° and 22.9°, measured at a temperature of about 20°C and using Cu-Kα radiation (wavelength λ= 1.5418 Å).

3. The crystalline form according to claims 1 to 2 **characterized by** a DTA/TGA trace showing an exotherm at 115°C and a melting endotherm at 151°C.

4. Crystalline form E of Entinostat having a x-ray powder diffraction pattern comprising peaks at 2θ values of 3.8°, 15.3° and 21.6°, measured at a temperature of about 20°C and using Cu-Kα radiation (wavelength λ= 1.5418 Å).

5. The crystalline form according to claim 4 further **characterized by** a x-ray powder diffraction pattern comprising 2θ values of 3.8°, 15.3°, 18.2°, 21.6°, 23.9° and 24.5°, measured at a temperature of about 20°C and using Cu-Kα radiation (wavelength λ= 1.5418 Å).

6. The crystalline form according to claims 4 to 5 **characterized by** a DTA/TGA trace showing an endotherm at 80°C and a melting endotherm at 155°C.

7. The crystalline forms according to claims 1 to 6 in substantially pure form.

8. Crystalline form A of Entinostat having a x-ray powder diffraction pattern (XRPD) comprising peaks at 2θ values of 18.4°, 18.8°, 19.1°, 20.9°, 22.6°, 26.4°, 26.7° and 27.2°, measured at a temperature of about 20°C and using Cu-Kα radiation (wavelength λ= 1.5418 Å), in substantially pure form.

9. The crystalline forms according to claims 1 to 8 including less than 10%, preferably less than 5%, more preferably less than 3%, most preferably less than 1% by weight of other crystalline forms.

10. A pharmaceutical composition comprising the crystalline form D or the crystalline form A of claims 1 to 3 or of claims 7 to 9 and a pharmaceutically acceptable carrier or diluent.

11. The crystalline form D or the crystalline form A of claims 1 to 3 or of claims 7 to 9, or the composition of claim 10 for use as a medicament, preferably for use in the treatment of breast cancer.

12. A process for the preparation of the crystalline form D of Entinostat of claims 1 to 3 comprising the step of drying crystalline form E of Entinostat.

13. A process for the preparation of the crystalline form E of Entinostat of claims 4 to 6 comprising the steps of suspending and/or dissolving Entinostat in a solvent comprising dioxane, preferably in pure dioxane, and then isolating crystalline form E.

14. A process for the preparation of crystalline form A of Entinostat, wherein form A is **characterized by** a x-ray powder diffraction pattern (XRPD) comprising peaks at 2θ values of 18.4°, 18.8°, 19.1°, 20.9°, 22.6°, 26.4°, 26.7° and 27.2°, measured at a temperature of about 20°C and using Cu-Kα radiation (wavelength λ= 1.5418 Å), comprising the steps of contacting the crystalline form D of Entinostat of claims 1 to 3 with a solvent selected from the group consisting of ethanol, acetone, acetonitrile, anisole, methyl ethyl ketone, t-butyl methyl ether, dimethylacetamide, dimethylformamide, ethyl acetate, isopropyl acetate, methanol, 2-methyltetrahydrofuran, methyl isobutyl ketone, toluene, tetrahydrofuran, 2-propanol / water mixtures, acetone / water mixtures and acetonitrile / water mixtures, and isolating crystalline form A.

15. Crystalline Entinostat obtainable by the process of claims 12 to 14.
